# EUROPEAN PATENT APPLICATION

(11) **EP 4 029 476 A1**
(43) Date of publication of application: **20.07.2022**
(21) Application number: 21839306.4
(22) Date of filing: 18.01.2021
(51) Int. Cl.: A61F 2/24

(54) **VALVE PROSTHETIC DEVICE FOR IMPLANTATION IN HEART**

(30) Priority: 23.11.2020 CN 202011321977; 23.11.2020 CN 202022725374 U
(71) Applicant: Jiangsu Trulive Medtech Co., Ltd, Rudong County Nantong Jiangsu 226400 (CN)
(72) Inventor: ZHAO, Jing, Shanghai 201206 (CN); YE, Jinhua, Shanghai 201206 (CN); YANG, Wei, Shanghai 201206 (CN)
(74) Representative: Argyma
(86) International application number: PCT/CN2021/072490
(87) International publication number: WO 2022/105055

(57) **Abstract**

A valve prosthesis apparatus for being implanted into a heart is provided. The valve prosthesis apparatus includes a valve body and a ventricle volume reduction member. The ventricle volume reduction member is fixed to the valve body by a connecting member. The ventricle volume reduction member is arranged by way of forming an isolation cavity for reducing the volume of the ventricle between the ventricle volume reduction member and an inner wall of an apex cordis.

## Description

### Field of the Invention

The present invention relates to the technical field of medical instruments, in particular to a valve prosthesis apparatus for being implanted into a heart.

### Description of the Prior Art

The heart contains four cavities, i.e., right atrium (RA), right ventricle (RV), left atrium (LA), and left ventricle (LV). During the entire cardiac cycle, pumping on the left and right sides of the heart usually occurs synchronously. A valve dividing the atrium from the ventricle is called an atrioventricular valve. The atrioventricular valve performs a "one-way valve" function to ensure the normal flow of blood in the cardiac cavity. The atrioventricular valve between the left atrium and the left ventricle is mitral valve, and the atrioventricular valve between the right atrium and the right ventricle is tricuspid valve. Pulmonary valve directs the blood flow to pulmonary artery and directs the blood flow from there to the lung. And then, the blood returns to the left atrium through the pulmonary vein. Aortic valve directs the blood flow through aorta and from there to the surrounding regions. Normally, there is no direct connection between the atriums or the ventricles. When the ventricle begins to inflate (dilate), both the aortic valve and the pulmonary valve close for preventing a regurgitation from the aorta to the ventricle. Shortly thereafter, the atrioventricular valve opens to allow unobstructed flow from the atrium into the corresponding ventricle. Shortly after the ventricular systole (i.e., the ventricular emptying) begins, the tricuspid valve and the mitral valve normally close, thereby forming a seal that prevents the regurgitation from the ventricle to the corresponding atrium.

When there is a problem with the atrioventricular valve, the atrioventricular valve cannot function properly, resulting in closing improperly. The atrioventricular valve is a complicated structure, which usually includes valve annulus, valve leaflets, chordae tendineae, and support structure. Each of the atriums is connected to the valve thereof by an atrium vestibule. The mitral valve has two leaflets, and the attachment or engagement between the corresponding surfaces of the valve leaflets facilitates providing closure or sealing to the valve, thereby preventing the blood from flowing in an incorrect direction. During the ventricular systole, a situation where the valve leaflets cannot be sealed is called a poor engagement, which causes the blood to flow reversely through the valve (i.e., the regurgitation). Transcatheter Mitral Valve Replacement (TMVR) is a method of using the intervention of catheters, wherein the prosthetic valve is compressed to the delivery system in vitro, sent to the mitral valve annulus of the human body along the blood vessel path or through the apex cordis, and then released and fixed to the mitral valve annulus for replacing the native valve. Compared with surgery, TMVR does not require extracorporeal circulation aids, causes small trauma, and allows the patients to recover quickly. Furthermore, the hemodynamic index of the patient improves significantly after the operation.

Research indicates that after the mitral valve regurgitation attacks, the load on the patient's left ventricle increases during the diastolic period, which in turn causes the left ventricle to enlarge. Then, the enlargement of the patient's left ventricle relatively reduces an ejection fraction, which in turn causes symptoms of heart failure such as tachypnea and fatigue. Therefore, developing a therapeutic apparatus that solves the mitral valve regurgitation and improves the enlargement of the left ventricle has great significance.

### SUMMARY OF THE INVENTION

The invention provides a valve prosthesis apparatus for being implanted into a heart, which may solve the above drawbacks in the prior art.

The technical solution of the invention is as follows:
A valve prosthesis apparatus for being implanted into a heart includes a valve body and a ventricle volume reduction member. The valve body includes a valve stent and prosthetic leaflets attached to the valve stent; the ventricle volume reduction member is fixed to the valve body by a connecting member, the ventricle volume reduction member being arranged by way of forming an isolation cavity for reducing a volume of the ventricle between the ventricle volume reduction member and an inner wall of an apex cordis; wherein after the prosthesis apparatus is implanted into the heart, the valve body is released at a native valve annulus for replacing a native valve to play a role in opening or closing a blood flow channel, and the ventricle volume reduction member is released at a left ventricle for improving a left ventricle enlargement caused by a mitral regurgitation.

The valve prosthesis apparatus for being implanted into a heart of the invention may be configured to replace a native valve and treat the mitral regurgitation caused by the atrioventricular valve lesions. Meanwhile, the ventricle volume reduction member isolates a part of the left ventricle by the isolation cavity formed between the ventricle volume reduction member and the apical inner wall, so that a volume of the left ventricle is restored to its normal size as much as possible, thereby decreasing a tension of the left ventricle wall, reducing an oxygen consumption of a cardiac muscle, enhancing a cardiac contractility and improving the functions of the heart (Laplace principle). Therefore, the valve prosthesis apparatus of the invention may be configured to treat the mitral regurgitation and the left ventricle enlargement caused by the mitral regurgitation and to improve heart failure, such that the valve prosthesis apparatus has important clinical significance.

In some embodiments, the ventricle volume reduction member is configured to be of an inverted cone structure, the ventricle volume reduction member comprises a large radial end portion, and the large radial end portion is located close to a side of the valve body; after the ventricle volume reduction member is released, the large radial end portion is adhered to the inner wall of the apex cordis, so that the isolation cavity is formed between the ventricle volume reduction member and the inner wall of the apex cordis for reducing the volume of the left ventricle and improving the enlargement of the left ventricle.

In some embodiments, the ventricle volume reduction member is configured to be of a disc-shaped structure, and the ventricle volume reduction member of the disc-shaped structure is adhered to the inner wall of the apex cordis radially to form the isolation cavity. Compared with the inverted cone structure, the ventricle volume reduction member is farther from the apex cordis after the ventricle volume reduction member of the disc-shaped structure is released, so that the inner wall of the apex cordis is avoided from contacting the ventricle volume reduction member when the heart contracts, thereby reducing the damage on the inner wall of the apex cordis. Further, the ventricle volume reduction member of the disc-shaped structure has a simpler structure, is easy to form, has a smaller size after being pressed and held, and is easy to release. In some embodiments, the ventricle volume reduction member includes a frame body and a frame skirt playing an isolating and sealing role, and the frame skirt covers on a surface of the frame body, so that the above isolation cavity is formed after the ventricle volume reduction member is released, wherein the skirt plays a sealing role.

In some embodiments, when the ventricle volume reduction member is configured to be of the disc-shaped structure, the frame body is formed by diverging several filamentous units outwards from the center, and wherein each of the filamentous units extends outwards radially by way of bending similarly.

In some embodiments, the ventricle volume reduction member further includes a sealing portion, the sealing portion is disposed on an end portion of the frame body close to the valve body, and the frame body is adhered to the inner wall of the apex cordis by the sealing portion to improve a sealing property of the isolation cavity. Meanwhile, the sealing portion also plays a certain buffering role to prevent the inner wall of the apex cordis from being damaged by the frame body.

In some embodiments, the frame body further includes a force dissipating apparatus, and the force dissipating apparatus is disposed on an end portion of the frame body away from the valve body, the frame body contacting the tissue by the force dissipating apparatus.

In some embodiments, an elastic buffer apparatus is further disposed between the force dissipating apparatus and the frame body. The force dissipating apparatus is disposed by way of increasing the contact area with the tissue, and the increased contact area allows an acting force between the force dissipating apparatus and the tissue to be more dispersed for preventing the force concentration and the tissue from being damaged. The elastic buffer apparatus plays a further buffering role, and the force dissipating apparatus and the elastic buffer apparatus are used to reduce the damage on the inner wall of the apex cordis when the heart contracts.

In some embodiments, the frame body is provided with a retention portion for fixing the connecting member, and the connecting member is fixed to the frame body by the retention portion.

In some embodiments, the prosthesis apparatus further includes an anchoring apparatus, and the anchoring apparatus provides an anchoring force for the ventricle volume reduction member and the valve body. The anchoring apparatus fixes the ventricle volume reduction member to the left ventricle to prevent the ventricle volume reduction member from being impacted by the blood or from moving in the process of heart contraction and dilation; in addition, the anchoring apparatus is further configured to anchor the valve body to prevent the valve body from displacing to the left ventricle due to the impact exerted by the blood.

In some embodiments, the anchoring apparatus includes a first anchoring portion, the first anchoring portion is disposed on an end portion of the ventricle volume reduction member away from the valve body, and the first anchoring portion provides the anchoring force by way of being attached to an outer wall of the apex cordis.

Compared with the conventional art, the present invention has the following beneficial effects:
First, the valve prosthesis apparatus for being implanted into a heart of the invention may be configured to replace a native valve and treat the mitral regurgitation caused by the atrioventricular valve lesions; meanwhile, the ventricle volume reduction member isolates a part of the left ventricle by the above isolation cavity formed between the ventricle volume reduction member and the apical inner wall, so that a volume of the left ventricle is restored to its normal size as much as possible, thereby decreasing a tension of the left ventricle wall, reducing an oxygen consumption of a cardiac muscle, enhancing a cardiac contractility and improving the functions of the heart (Laplace principle); therefore, the valve prosthesis apparatus of the invention can be configured to treat the mitral regurgitation while treating the left ventricle enlargement caused by the mitral regurgitation, and to improve the heart failure, with important clinical significance.
Second, according to the valve prosthesis apparatus for being implanted into a heart of the invention, the ventricle volume reduction member may be configured to be of the inverted cone structure with a large radial end portion adhered to the ventricle inner wall to form the isolation cavity; when the ventricle volume reduction member is configured to be of the disc-shaped structure, compared with the inverted cone structure, the ventricle volume reduction member of the disc-shaped structure is farther from the apex cordis after being released, so that the inner wall of the apex cordis is avoided from contacting the ventricle volume reduction member when the heart contracts, thereby reducing the damage on the inner wall of the apex cordis; moreover, the ventricle volume reduction member of the disc-shaped structure has a simpler structure, is easy to form, has a smaller size after being pressed and held, and is easy to release.

Certainly, any one product for implementing the present invention is unnecessary to achieve all the above advantages at the same time.

### BRIEF DESCRIPTION OF THE DRAWINGS

Fig. 1 is an integral structural diagram of a valve prosthesis apparatus according to Embodiment 1 of the invention;
Fig. 2 is an integral structural diagram of a ventricle volume reduction member according to Embodiment 1 of the invention;
Fig. 3 is one structural diagram of implanting the valve prosthesis apparatus into a heart according to Embodiment 1 of the invention;
Fig. 4 is the other structural diagram of implanting the valve prosthesis apparatus into a heart according to Embodiment 1 of the invention;
Fig. 5 is a structural diagram after the valve prosthesis apparatus is released according to Embodiment 1 of the invention;
Fig. 6 is a front structural diagram of the ventricle volume reduction member according to Embodiment 2 of the invention;
Fig. 7 is an integral structural diagram of releasing the valve prosthesis apparatus to the heart according to Embodiment 2 of the invention;
Fig. 8 is an integral structural diagram of the ventricle volume reduction member according to Embodiment 3 of the invention;
Fig. 9 is one structural diagram of implanting the valve prosthesis apparatus into a heart according to Embodiment 3 of the invention;
Fig. 10 is a structural diagram of beginning to release the valve prosthesis apparatus according to Embodiment 3 of the invention;
Fig. 11 is a structural diagram of a release process of the valve prosthesis apparatus is released according to Embodiment 3 of the invention;
Fig. 12 is a structural diagram of completing releasing the valve prosthesis apparatus according to Embodiment 3 of the invention.

Reference for numerals: valve body 100; valve stent 110; ventricle volume reduction member 200; connecting member 300; isolation cavity 500; frame body 201; frame skirt 202; elastic buffer apparatus 203; force dissipating apparatus 204; retention portion 205; large radial end portion 210; first anchoring portion 400; first region 101; second region 102; third region 103; sealing portion 206; second anchoring portion 207; delivery system 600; filamentous unit 2011.

### DESCRIPTION OF THE PREFERRED EMBODIMENTS

In the description of the invention, it should be noted that "heart valve prosthesis apparatus", "valve prosthesis apparatus", and "prosthesis apparatus" have the same meaning, and are "the valve prosthesis apparatus for being implanted into a heart" of the invention.

In the description of the invention, it should be noted that the "axial direction" refers to a direction from the valve body to the ventricle volume reduction member, and the "radial direction" refers to a direction perpendicular to the axial direction.

In the description of the present invention, it should be noted that orientations or position relationships indicated by terms "center", "upper", "lower", "left", "right", "vertical", "horizontal", "inside", "outside" and the like are orientations or position relationships shown in the drawings, and these terms are merely for facilitating description of the present invention and simplifying the description, but not for indicating or implying that the mentioned apparatus or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

As used in the specification, singular forms "a/an," "one," and "the/that" include plural objects, unless otherwise explicitly stated. As used in the specification, the term "or" is usually used to include the meaning of "and/or", unless otherwise expressly stated.

The following further describes the present invention in combination with specific embodiments.

### Embodiment 1

The embodiment provides a valve prosthesis apparatus for being implanted into a heart including a valve body 100 and a ventricle volume reduction member 200. The valve body 100 includes a valve stent 110 and prosthetic leaflets attached to the valve stent 110; the ventricle volume reduction member 200 is fixed to the valve body 100 by a connecting member 300, the ventricle volume reduction member 200 being arranged by way of forming an isolation cavity 500 for reducing a volume of the ventricle between the ventricle volume reduction member and an inner wall of an apex cordis; wherein after the prosthesis apparatus is implanted into the heart, the valve body 100 is released at a native valve annulus for replacing a native valve to play a role in opening or closing a blood channel, and the ventricle volume reduction member 200 is released at a left ventricle for improving a left ventricle enlargement caused by a mitral regurgitation.

In the prior art, the heart valve is only used to replace the native valve and treat lesions of the atrioventricular valve without considering the problems of increased load and enlargement of left ventricle caused by the mitral valve regurgitation during the diastolic period of the left ventricle. The prosthesis apparatus of the embodiment may be used to treat the mitral regurgitation caused by the lesions of the atrioventricular valve and the ventricle enlargement caused by the mitral valve regurgitation simultaneously, with an important clinical significance. The valve body 100 is configured to replace the native valve, and the isolation cavity 500 formed between the ventricle volume reduction member 200 and the inner wall of the apex cordis isolates a part of the left ventricle, so that a volume of the left ventricle is restored to its normal size as much as possible, thereby decreasing a tension of the left ventricle wall, reducing an oxygen consumption of a cardiac muscle, enhancing a cardiac contractility and further improving the functions of the heart (Laplace principle).

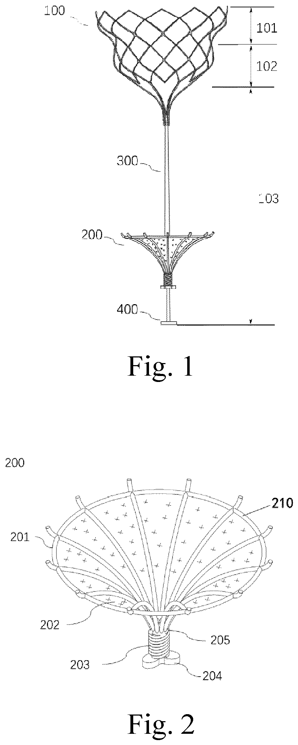

With reference to Figs. 1 to 5, structural diagrams of the valve prosthesis apparatus in the embodiment are shown.

In the embodiment, the ventricle volume reduction member 200 is configured to be of an inverted cone structure, and the ventricle volume reduction member 200 includes a large radial end portion 210, wherein the large radial end portion 210 is located a side close to the valve body 100, and wherein after the ventricle volume reduction member 200 is released, the large radial end portion 210 is adhered to the inner wall of the apex cordis so that the isolation cavity 500 is formed between the ventricle volume reduction member 200 and the inner wall of the apex cordis. The inverted cone structure allows a side direction of the ventricle volume reduction member 200 may form the above isolation cavity 500 together with the inner wall of the apex cordis after the ventricle volume reduction member is released, so as to further reduce the volume of the left ventricle and improve the problem of heart failure caused by the left ventricle enlargement.

Specifically, in the embodiment, the ventricle volume reduction member 200 includes a frame body 201 and a frame skirt 202, playing an isolating and sealing role. The frame skirt 202 covers a surface of the frame body 201 for the sealing so that an enclosed isolation cavity 500 is formed after the ventricle volume reduction member 200 is released. The frame skirt 202 may be of a single-layered structure or a double-layered structure; the frame skirt 202 may cover an inner surface of the frame body 201, or is disposed on an outer surface of the frame body 201, or is disposed on the inner surface and the outer surface of the frame body 201 simultaneously, the frame skirt 202 mainly playing a sealing role. An end portion of the frame body 201 close to the valve body 100 is the above large radial end portion 210, and a circumferential size of the frame body 201 reduces gradually in a direction from the valve body 100 to the ventricle volume reduction member 200.

Further, the frame body 201 of the embodiment further includes a force dissipating apparatus 204. The force dissipating apparatus 204 is disposed at an end portion of the frame body 201 away from the valve body 100. Further, an elastic buffer apparatus 203 is further disposed between the force dissipating apparatus 204 and the frame body 201.

The force dissipating apparatus 204 is disposed by way of increasing the contact area with the tissue, e.g., configured as a laminar structure with smooth edges, and the increased contact area allows an acting force between the force dissipating apparatus 204 and the tissue when the former contacts the latter to be more dispersed for preventing the force concentration and the tissue from being damaged. The elastic buffer apparatus 203 may be configured to be of a spiral-shaped structure or a spring-shaped structure; the elastic buffer apparatus 203 plays a further buffering role, and the force dissipating apparatus 204 and the elastic buffer apparatus 203 are configured to reduce the damage on the inner wall of the apex cordis exerted by the end portion of the frame body 201 when the heart contracts.

In the embodiment, the frame body 201 is provided with a retention portion 205 configured to fix a connecting member 300. The retention portion 205 is formed as a channel structure for the connecting member 300 to pass through, and to be fixedly connected with the frame body 201. Specifically, the other end of the connecting member 300 is fixed to an end portion of the valve stent 110.

Further, the ventricle volume reduction member 200 of the embodiment further includes an anchoring apparatus, and the anchoring apparatus provides the anchoring force to the ventricle volume reduction member 200 and the valve body 100. The anchoring apparatus fixes the ventricle volume reduction member 200 to the left ventricle to prevent the ventricle volume reduction member from being impacted by the blood or from being forced to move in the process of heart contraction and dilation; in addition, the anchoring apparatus is further configured to anchor the valve body 100 to prevent the valve body 100 from displacing to the left ventricle due to the impact exerted by the blood. Preferably, the ventricle volume reduction member 200 is fixed to a region of the apex cordis of the left ventricle, and the region of the apex cordis has no chordae tendineae and papillary muscle so that it is relatively easy to anchor.

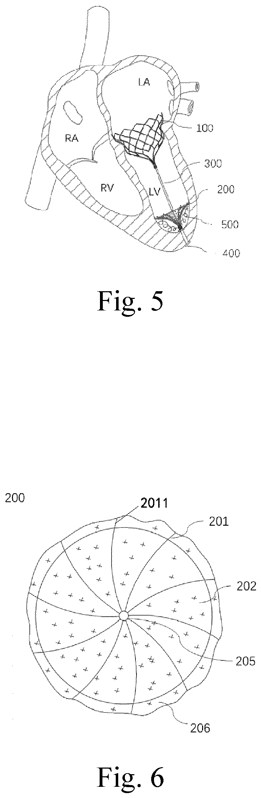

With reference to Figs. 1 and 5, in the embodiment, the anchoring apparatus includes a first anchoring portion 400, the first anchoring portion 400 is disposed on an end portion of the ventricle volume reduction member 200 away from the valve body 100, and the first anchoring portion 400 provides the anchoring force by way of being attached to an outer wall of the apex cordis after being released. This structure allows the prosthesis apparatus of the embodiment to be delivered to the mitral valve via the apex cordis.

With continued reference to Figs. 1, 3, and 5, the valve stent 110 of the embodiment may be formed as a structure with openings at both ends, which has structures with a cross section of a circle shape, a D shape, a flower shape, or a combination thereof. The valve stent 110 may be divided into a first region 101, a second region 102, and a third region 103 axially. After the valve body 100 is implanted into a human body, the first region 101 is adhered to a native mitral valve annulus of the heart to prevent the prosthesis valve from falling from the left atrium into the left ventricle, and the second region 102 is configured to carry the prosthetic valve leaflets while playing a certain anchoring and sealing role by being supported on the tissue radially. An end portion of the valve stent 110 close to the ventricle volume reduction member 200 includes several end nodes, and the end nodes further extend to form a structure fixedly connected with the connecting member 300. The third region 103 is located in the ventricle and includes the ventricle volume reduction member 200, the connecting member 300, and the anchoring apparatus.

The valve stent 110 may provide several functions to the valve body 100, which include being used as a body structure of the valve, carrying an internal prosthetic valve leaflet, being used as a seal for inhibiting the circumferential leakage of valve between the valve body 100 and the native valve, and being used as a connecting structure (e.g., two end portions of the valve stent 110 in the axial direction are provided with a hanging tab or a fixing tab) with the delivery system 600, and so on. Specifically, the valve stent 110 is of a frame structure formed by arranging several enclosed geometric units, wherein the enclosed geometric unit includes a triangle shape, a diamond shape, a droplet shape, a heart shape, and so on. The valve stent 110 may be manufactured by a weaving process or a cutting process, and optionally may be made from nickel-titanium alloys or other biocompatible materials with shape memory properties or from an elastic or malleable deformable material such as a balloon-expandable material.

The valve body 100 of the embodiment further includes a skirt covering the surface of the valve stent 110. The skirt may be of the single-layered structure, or may be of an internal-external double-layered structure, which may select materials such as polyester fabrics made by knitting, tatting and weaving, PTFE and ePTFE, so as to mainly play a sealing role for preventing regurgitation.

The prosthetic valve leaflet dynamically switches between the two states of an opening state and a closing state, with the number that is the same as or different from that of the native valve leaflets. The prosthetic valve leaflet closes tightly or converges by way of sealing and abutting when being in the closing state. The prosthetic valve leaflet may be made from any suitable material or a combination of materials. In some embodiments, the prosthetic valve leaflet may be prepared by biological tissues such as chemically stable tissues from the heart valves of animals such as pigs or pericardium tissues such as cattle (bovine pericardium tissues) or sheep (ovine pericardium tissues) or pigs (porcine pericardium tissues) or horses (equine pericardium tissues), preferably the cattle pericardium tissues. In some embodiments, the prosthetic valve leaflet may also be made from small intestinal submucosa tissues. Further, synthetic material may also be used for the prosthetic valve leaflet, for example, expanded PTFE or polyester. Optionally, the material further includes thermoplastic polyurethane polyester, polyether urethane, segmented polyether urethane, silicone polyether urethane, silicone-polyurethane polyester, and ultra-high molecular weight polyethylene. Moreover, the biocompatible polymer may also be used for the prosthetic valve leaflet, which optionally may include polyolefin, elastomer, polyethylene glycol, polyethersulfone, polysulfone, polyvinylpyrrolidone, polyvinyl chloride, other fluorine-containing polymers, silicone polyester, siloxane polymers and/or olimers, and/or polycaprolactone, and block copolymers thereof. Optionally, the prosthetic valve leaflet further has a surface treated by (or reacted with) anticoagulants, and the anticoagulant includes but is not limited to heparinized polymers.

In the embodiment, the connecting member 300 provides a traction force to the valve body 100, so as to prevent the stent from being displaced to the left atrium due to the impact exerted by the blood when the heart contracts. The connecting member 300 may be made from, e.g., a biocompatible polymer material, which includes but is not limited to ultra-high molecular weight polyethylene (UHMWPE), polytetrafluoroethylene, and so on. The connecting member 300 may be elastic-free so as to provide a more firm anchoring force; the connecting member 300 may also be elastic so that a higher degree of stretch compliance is provided during the cardiac cycle. Optionally, the connecting member 300 may be made from a bioabsorbable material, and thus provides temporary fixation until the endothelialization between the valve body 100 and the tissue is enough to provide the anchoring force of the valve body.

In the embodiment, the frame body 201 may be made by the weaving process or the cutting process, and a material of the frame body 201 is the same as or different from the material of the valve stent 110. Optionally, the frame body 201 may be made from nickel-titanium alloys or other biocompatible materials with shape memory properties, or from an elastic or malleable deformable material such as a balloon-expandable material. A material of the frame skirt 202 is the same as or different from a material of the skirt of the valve body 100, and the frame skirt 202 may select materials such as PTFE, ePTFE, or polyester fabrics made by knitting, tatting, or weaving.

Specifically, in the embodiment, the skirt and the frame skirt 202 may be configured by way of stitching or covering; the connecting member 300 may be fixed with the valve stent 100 and the frame body 201 by way of stitching, welding, and so on. The above-mentioned ways should not limit the protection scope of the invention and may be selected according to actual processes.

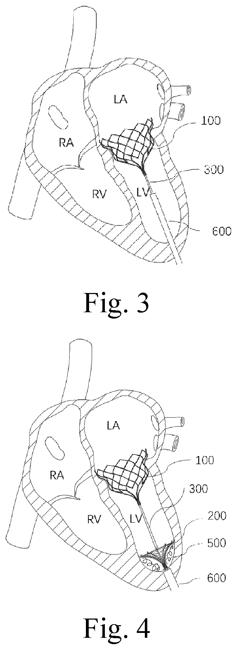

In the embodiment, the valve prosthesis apparatus for being implanted into a heart has the following implanting process: with reference to Figs. 3, 4, and 5, the prosthesis apparatus is delivered to the lesions of the mitral valve via the apex cordis by a delivery system 600. At first, the valve body 100 is released. Then, after the position of the valve stent 110 is adjusted, the ventricle volume reduction member 200 is released. Finally, after the position is adjusted, the first anchoring portion 400 is released for completing the release.

### Embodiment 2

The embodiment provides a valve prosthesis apparatus for being implanted into a heart. The prosthesis apparatus of the embodiment is similar to the prosthesis apparatus of Embodiment 1 except for the structure of the ventricle volume reduction member 200. In the embodiment, the ventricle volume reduction member 200 is configured to be of a disc-shaped structure, and the ventricle volume reduction member 200 of the disc-shaped structure is adhered to the inner wall of the apex cordis radially to form the isolation cavity 500.

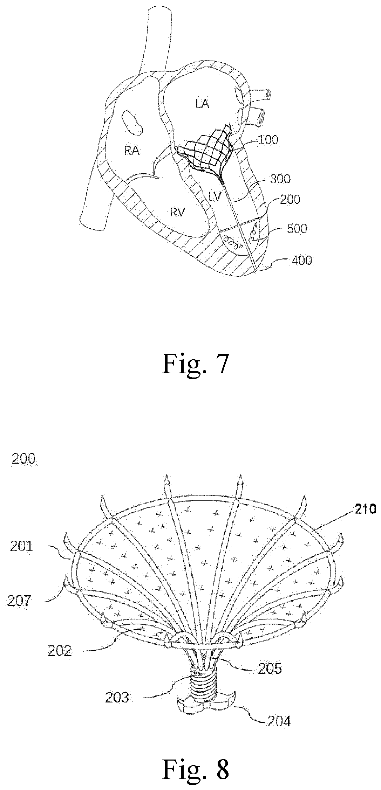

With reference to Figs. 6 and 7, structural diagrams of the valve prosthesis apparatus in the embodiment are shown.

Compared with the inverted cone structure, the ventricle volume reduction member 200 of the embodiment is farther from the apex cordis after being released, so that the inner wall of the apex cordis is avoided from contacting the ventricle volume reduction member 200 when the heart contracts, thereby reducing the damage on the inner wall of the apex cordis. Further, the ventricle volume reduction member of the disc-shaped structure in the embodiment has a simpler structure, is easy to form, has a smaller size after being pressed and held, and is easy to release.

Specifically, when the ventricle volume reduction member of the embodiment is formed, the frame body 201 is formed by diverging several filamentous units 2011 outwards from the center. Each of the filamentous units 2011 extends at a pre-set bending curvature in the radial direction. For example, each of the filamentous units 2011 extends in an arc shape, an S shape, or a wave shape. Thereby, when the heart contracts, the frame body 201 of the embodiment has a certain stiffness to be capable of withstanding the squeezing force for conformably deforming and restoring from deformation; in addition, the frame body 201 is capable of being adhered to the inner wall of the apex cordis to maintain the above isolation cavity 500. Specifically, in the embodiment, each of the filamentous units 2011 extends by the same bending way, and each of the filamentous units has the same bending curvature, so that the frame body 201 has enough stiffness.

The ventricle volume reduction member 200 further includes a sealing portion 206. The sealing portion 206 is disposed in the circumferential direction of the frame body 201, and the frame body 201 is adhered to the inner wall of the apex cordis by the sealing portion 206 to improve a sealing property of the isolation cavity 500. Specifically, the sealing portion 206 extends outwards in the circumferential direction from the frame body 201 so that the frame body 201 well adheres to the inner wall of the apex cordis; meanwhile, the sealing portion 206 also plays a certain buffering role to reduce the damage on the inner wall of the apex cordis exerted by the frame body 201 when the heart contracts. A material of the sealing portion 206 may select materials such as PTFE, ePTFE, and polyester fabrics made by knitting, tatting, or weaving. The sealing portion is fixed to the frame body 201 by way of stitching, covering, and so on.

### Embodiment 3

The embodiment provides a valve prosthesis apparatus for being implanted into a heart. The prosthesis apparatus of the embodiment is similar to the prosthesis apparatus of Embodiment 1, wherein the ventricle volume reduction member 200 is of an inverted cone shape. Embodiment 3 differs from Embodiment 1 in the structure of the anchoring apparatus. In Embodiment 3, the anchoring apparatus includes a second anchoring portion 207, wherein the second anchoring porting 207 is disposed in the circumferential direction of the ventricle volume reduction member 200, and the second anchoring portion 207 provides the anchoring force by way of piercing into the inner wall of the apex cordis instead of being attached to a ventricle outer wall in Embodiment 1.

Specifically, with reference to Fig. 8, the large radial end portion 210 of the frame body 201 contacts the ventricle inner wall, and the force dissipating apparatus 204 of the frame body 201 contacts the apex cordis; therefore, the second anchoring portion 207 may be distributed in a circumferential direction of the large radial end portion 210 uniformly, or may be distributed on the free end of the force dissipating apparatus 204. The second anchoring portion 207 may pierce into the inner wall of the apex cordis in the form of the anchoring needle, or the second anchoring portion 207 may also provide the anchoring force by way of clamping the tissue.

The prosthesis apparatus of the embodiment may treat the mitral valve regurgitation and improve the heart failure simultaneously. Since the anchoring apparatus provides the anchoring force by way of being attached to the ventricle inner wall, the prosthesis apparatus of the embodiment may be released via an interatrial septum, which does not require thoracotomy as compared to the way of being implanted via the apex cordis and hence may reduce the trauma of the patient. Moreover, the ventricle volume reduction member 200 of the embodiment not only plays a role in reducing the volume of the ventricle but also provides the anchoring force, and the second anchoring portion 207 is attached to the ventricle inner wall to provide the anchoring force, so as to change the traditional anchoring way of clamping the valve leaflets or capturing the valve leaflets, thereby making the anchoring firm and being unable to stretch the chordae tendineae or damage the valve leaflets.

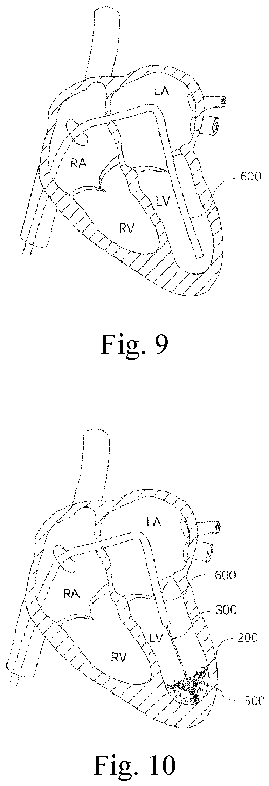

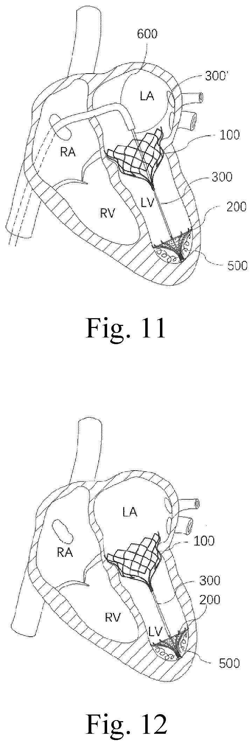

In the embodiment, the valve prosthesis apparatus for being implanted into a heart has the following implanting process:
with reference to Figs. 9 to 12, the delivery catheter 600 enters the right atrium via an inferior vena cava and then passes the interatrial septum and the mitral valve to reach near the apex cordis; the ventricle volume reduction member 200 is released to the apex cordis first, and the second anchoring portion 207 on the ventricle volume reduction member 200 is connected with the inner wall of the apex cordis to form the anchoring relationship after being released out of the delivery system; subsequently, the connecting member 300 and the valve body 100 are continuously released out, and after the valve body 100 is completely released out, a tension of the connecting member 300 is adjusted to reach an optimal sealing effect; then, the connecting member 300 is fixed and cut, followed by withdrawing the delivery system to complete the release.

The valve prosthesis apparatus of the invention can be configured to replace a native valve and treat the mitral regurgitation caused by the atrioventricular valve lesions. Meanwhile, the ventricle volume reduction member isolates a part of the left ventricle by the isolation cavity formed between the ventricle volume reduction member and the apical inner wall, so that a volume of the left ventricle is restored to its normal size as much as possible, thereby decreasing a tension of the left ventricle wall, reducing an oxygen consumption of a cardiac muscle, enhancing a cardiac contractility and improving the functions of the heart. Therefore, the valve prosthesis apparatus of the invention can be configured to treat the mitral regurgitation while treating the left ventricle enlargement caused by the mitral regurgitation, and to improve the heart failure, with important clinical significance.

The above disclosure is only the preferred embodiment of the invention, and the preferred embodiment does not describe all the details in detail. It should be understood that these embodiments are only used for illustrating the invention but not for limiting the scope thereof. The technical characteristics in the different embodiments above can be combined arbitrarily without conflict.

The present invention selects and specifically describes the embodiments with the purpose of better explain the principle and practical use of the present invention, such that a person skilled in the art can well utilize the present invention. The present invention is merely limited by the appended claims and the scope and equivalents thereof. In practical application, the improvements and adjustments made by those skilled in the art according to the present invention still fall within the scope of protection of the present invention.

## Claims

1. A valve prosthesis apparatus for being implanted into a heart, comprising:
a valve body, comprising a valve stent and prosthetic leaflets attached to the valve stent;
a ventricle volume reduction member, fixed to the valve body by a connecting member, the ventricle volume reduction member being arranged by way of forming an isolation cavity for reducing a volume of the ventricle between the ventricle volume reduction member and an inner wall of an apex cordis;
wherein after the prosthesis apparatus is implanted into the heart, the valve body is released at a native valve annulus for replacing a native valve to play a role in opening or closing a blood flow channel, and the ventricle volume reduction member is released at a left ventricle for improving a left ventricle enlargement caused by a mitral regurgitation.

2. The valve prosthesis apparatus for being implanted into a heart according to claim 1, wherein the ventricle volume reduction member is configured to be of an inverted cone structure, the ventricle volume reduction member comprises a large radial end portion, and the large radial end portion is located close to a side of the valve body;
after the ventricle volume reduction member is released, the large radial end portion is adhered to the inner wall of the apex cordis, so that the isolation cavity is formed between the ventricle volume reduction member and the inner wall of the apex cordis.

3. The valve prosthesis apparatus for being implanted into a heart according to claim 1, wherein the ventricle volume reduction member is configured to be of a disc-shaped structure, and the ventricle volume reduction member of the disc-shaped structure is adhered to the inner wall of the apex cordis radially to form the isolation cavity.

4. The valve prosthesis apparatus for being implanted into a heart according to claim 1, 2, or 3, wherein the ventricle volume reduction member comprises a frame body and a frame skirt playing an isolating and sealing role, and the frame skirt covers on a surface of the frame body, so that the above isolation cavity is formed after the ventricle volume reduction member is released.

5. The valve prosthesis apparatus for being implanted into a heart according to claim 4, wherein when the ventricle volume reduction member is configured to be of the disc-shaped structure, the frame body is formed by diverging several filamentous units outwards from the center, and wherein each of the filamentous units extends outwards radially by way of bending similarly.

6. The valve prosthesis apparatus for being implanted into a heart according to claim 4, wherein the ventricle volume reduction member further comprises a sealing portion, the sealing portion is disposed on an end portion of the frame body close to the valve body, and the frame body is adhered to the inner wall of the apex cordis by the sealing portion to improve a sealing property of the isolation cavity.

7. The valve prosthesis apparatus for being implanted into a heart according to claim 4, wherein the frame body further comprises a force dissipating apparatus, and the force dissipating apparatus is disposed on an end portion of the frame body away from the valve body.

8. The valve prosthesis apparatus for being implanted into a heart according to claim 7, wherein an elastic buffer apparatus is further disposed between the force dissipating apparatus and the frame body.

9. The valve prosthesis apparatus for being implanted into a heart according to claim 4, wherein the frame body is provided with a retention portion for fixing the connecting member.

10. The valve prosthesis apparatus for being implanted into a heart according to claim 1, wherein the prosthesis apparatus further comprises an anchoring apparatus, and the anchoring apparatus provides an anchoring force for the ventricle volume reduction member and the valve body.

11. The valve prosthesis apparatus for being implanted into a heart according to claim 10, wherein the anchoring apparatus comprises a first anchoring portion, the first anchoring portion is disposed on an end portion of the ventricle volume reduction member away from the valve body, and the first anchoring portion provides the anchoring force by way of being attached to an outer wall of the apex cordis.
